# EUROPEAN PATENT APPLICATION

(11) **EP 4 137 504 A1**
(43) Date of publication of application: **22.02.2023**
(21) Application number: 21788271.1
(22) Date of filing: 12.04.2021
(51) Int. Cl.: C07K 7/06, A61K 9/51, A61K 47/42

(54) **NOVEL ALPHA-HELICAL DOUBLE-SIDED CELL PENETRATING PEPTIDE AND USE THEREOF**

(30) Priority: 14.04.2020 KR 20200045454
(71) Applicant: Camp Therapeutics Inc., Seoul 08826 (KR)
(72) Inventor: YU, Jae-Hoon, Seongnam-si, Gyeonggi-do 13531 (KR); HYUN, Soon-Sil, Seoul 08826 (KR); CHO, Jane, Seoul 08826 (KR); KIM, Dong-Woo, Seoul 08826 (KR)
(74) Representative: Schnappauf, Georg
(86) International application number: PCT/KR2021/004592
(87) International publication number: WO 2021/210868

(57) **Abstract**

The present disclosure relates to a novel amphipathic, alpha-helical cell-penetrating peptide and uses thereof. The cell-penetrating peptide of the present disclosure has a nanoparticle self-association characteristic and penetrates into eukaryotic cells at a nanomolar concentration, which allows the peptide to be advantageously used for delivery, into cells, of a drug or the like that is difficult to penetrate into the cells.

## Description

### Technical Field

The present disclosure relates to a novel amphipathic, alpha-helical cell-penetrating peptide and uses thereof. The cell-penetrating peptide of the present disclosure has a nanoparticle self-association characteristic and penetrates into eukaryotic cells at a nanomolar concentration, which allows the peptide to be advantageously used for delivery, into cells, of a drug or the like that is difficult to penetrate into the cells.

### Background Art

Cell-penetrating peptides ("CPPs") are used for the purpose of delivering modulators, in particular, biological drugs, which are difficult to penetrate into cells. Here, the modulator may be covalently linked to CPP or form a complex therewith. Use of CPPs makes it possible to deliver modulators, which could have been delivered into cells only at high concentrations in a case of being used alone, even at low concentrations.

Dozens of CPPs have been developed since the Tat peptide was reported as the first CPP in the 1980s, and it has been found that these CPPs can be used to deliver small-molecule modulators, fluorescent substances, or the like into cells. However, there are very few cases where CPPs are actually used to deliver therapeutic agents for diseases into cells to improve their therapeutic effect. This is because CPPs, which have been known so far, exhibit cell-penetrating ability at a relatively high (micromolar or higher) concentration, and this concentration is very high as compared with a therapeutic agent that is capable of exhibiting a nanomolar effect in a living body (or cell). That is, in a case where a therapeutic agent is delivered into cells at a high (micromolar or higher) concentration, severe toxicity may be caused by the therapeutic agent that typically has nanomolar binding affinity. Moreover, since CPPs themselves may be toxic, there have been several problems in commercial application of CPPs *in vivo* at a micromolar or higher concentration.

In the previous study, the present inventors found that in a case where a peptide containing a covalent linkage site at a specific amino acid position was made into a dimer and the peptide was allowed to have a maximized alpha-helical content, the peptide had remarkably increased cell-penetrating ability and was able to be delivered into cells even at a nanomolar concentration (WO 2015/057009). In particular, the present inventors found that the amphipathic alpha-helical peptide (LK-1) composed of Leu and Lys became LK-2 in a case where leucine was substituted with cysteine at the positions i and i+7 of the hydrophobic surface; LK-2 was made into a dimeric peptide (LK-3) by forming two disulfide bonds under a condition where cysteine is oxidized; and LK-3 was characterized as having cell-penetrating ability even at several nanomolar concentrations. However, LK-3 is a dimeric bundle peptide whose monomer consists of a total of 16 amino acids and is not suitable for commercial use due to its slow manufacturing process, unsatisfactory cytotoxicity, and the like.

There is still a need for CPPs having minimal cytotoxicity while exhibiting excellent cell-penetrating ability.

### Disclosure of Invention

### Technical Problem

The present disclosure intends to provide a commercially utilizable cell-penetrating peptide in consideration of cell-penetrating ability, simplified manufacturing process, material properties such as solubility in water of its dimeric peptide, and cytotoxicity.

The present disclosure also intends to provide a platform technology capable of delivering a biologically active substance, such as a therapeutic drug, into cells using the cell-penetrating peptide.

### Solution to Problem

The present disclosure provides a dimeric peptide, comprising, as a monomer, a peptide represented by Formula 1.

<Formula 1> X₁X₂X₃X₄X₅X₆X₇X₈X₉X₁₀

In the formula,
X₁, X₄, X₅, and X₈ are each independently a hydrophobic amino acid,
X₃, X₆, X₇, and X₁₀ are each independently a hydrophilic amino acid, and
X₂ and X₉ are each independently an amino acid that forms a bond so that each monomeric peptide may be linked to each other at at least one position of X₂ and X₉ to form a dimeric peptide.

The present disclosure also provides a pharmaceutical composition comprising the dimeric peptide and a biologically active substance.

### Advantageous Effects of Invention

The amphipathic, alpha-helical peptide according to the present disclosure is characterized by nanoparticle self-association and exhibits high cell-penetrating ability. Accordingly, the peptide of the present disclosure not only can effectively deliver various biologically active substances into cells but also exhibit minimized cytotoxicity after cell penetration, which allows the peptide to be advantageously used in the field of prevention or treatment of diseases.

In addition, the dimeric peptide of the present disclosure is economically useful and is suitable for commercial use due to being rapidly produced from its monomer.

### Brief Description of Drawings

FIG. 1 illustrates rates, at which dimeric cell-penetrating peptides are formed (as measured by changes in monomer concentration), and rate constant k values, depending on changes in monomer length.
FIG. 2A illustrates rates at which various dimeric cell-penetrating peptides are formed (as measured by changes in monomer concentration). In respective graphs, the monomer concentration (%) at 24 hours is lowest in the order of FK10, LK10, FR10, IR10, LR10, IK10, NpgR10, LH10, VK10, FH10, and NpgK10 (however, the graphs for FR10, IR10 and LR10 almost overlap after 8 hours).
FIG. 2B illustrates a schematic structure of a dimeric peptide having a nanoparticle self-association characteristic.
FIG. 3 illustrates schematics in which spacing between monomers in each dimer is modified: 16-mer (LK-3) dimers (FIG. 3A); 10-mer dimers (FIG. 3B).

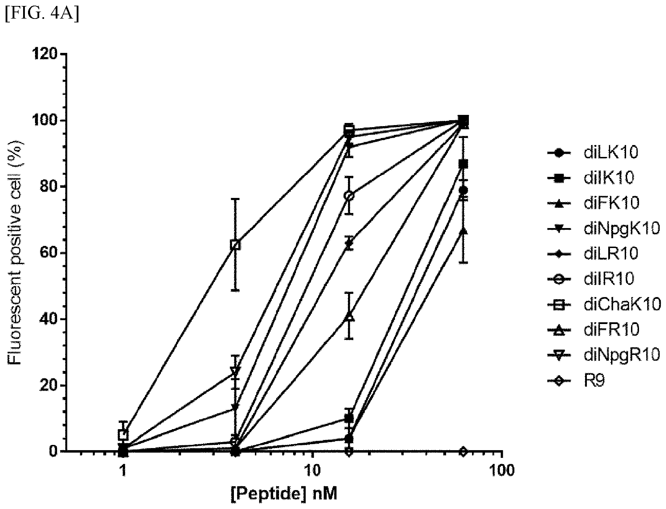

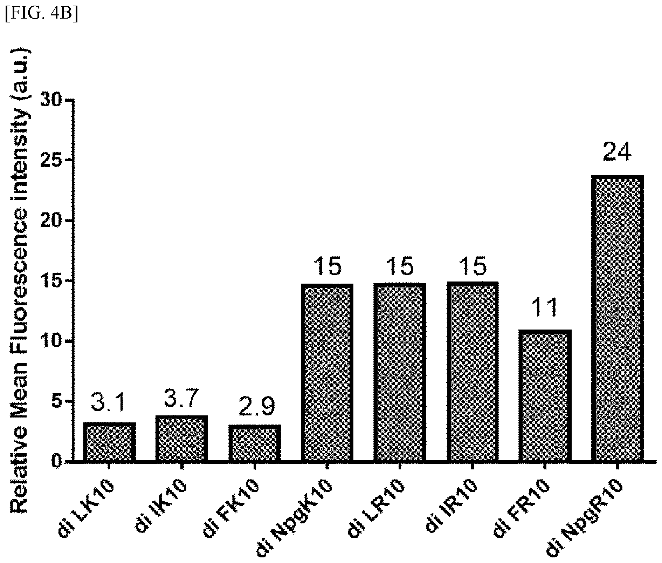

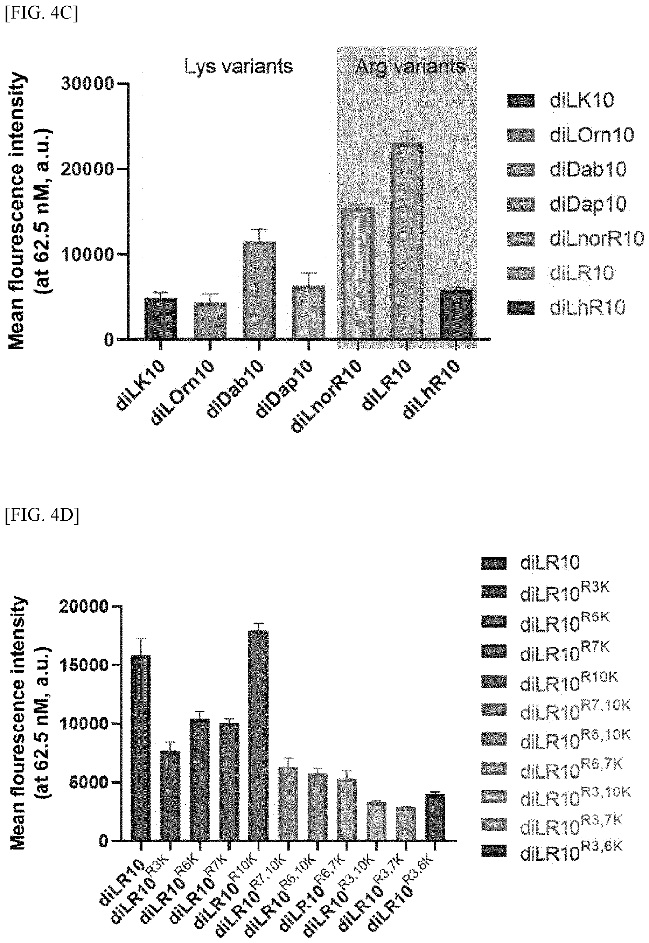

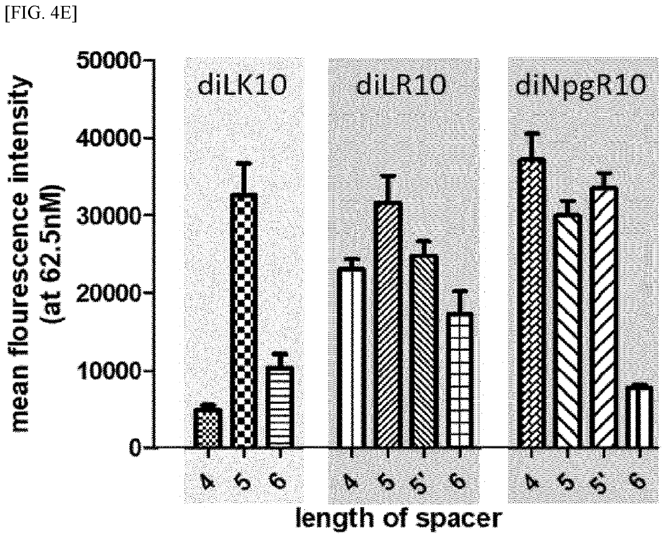

FIG. 4 illustrates cell-penetrating ability of the dimeric peptides of the present disclosure. FIG. 4A illustrates percentage (%) of cells for which fluorescence is detected. At a peptide concentration of about 15 nM, the cell percentage (%) is lowest in the order of R9 (control), diFK10, diLK10, diIK10, diFR10, diLR10, diIR10, diNpgK10, diNpgR10, and diChaK10. FIGS. 4B to 4E illustrate results obtained by comparing mean fluorescence intensity at the same concentration (62.5 nM). FIG. 4B illustrates relative fluorescence intensity obtained in a case where the fluorescence intensity of diChaK10 is set to 1.
FIG. 5 illustrates cytotoxicity of the dimeric peptides of the present disclosure (as measured by cell viability).
FIG. 6A illustrates correlation between cell-penetrating ability and cytotoxicity of the dimeric peptides of the present disclosure.
FIG. 6B illustrates nanoparticle self-association tendency of the dimeric peptides of the present disclosure as relative changes in retention time depending on temperatures obtained through reverse phase chromatography (ΔRt value at the highest temperature in each graph is lowest in the order of AcLR10 mono, diFR10, diLR10, LK03, helix A, diLK10 (almost overlapping with helix A), diNpgR10, and diNpgK10).
FIG. 6C illustrates correlation between nanoparticle self-association tendency and cytotoxicity of the dimeric peptides of the present disclosure.
FIG. 7 illustrates synthesis of parallel or antiparallel diLK10 dimers and cell-penetrating ability of the respective dimers.
FIG. 8 illustrates results obtained by identifying level of intracellular delivery of cyclosporin with cell-penetrating peptides in a case where the cyclosporin is fluorescently-labeled and non-covalently bound to the cell-penetrating peptide.
FIG. 9 illustrates intracellular delivery of an antibody using cell-penetrating peptides, each of which is conjugated with streptavidin and biotin.

### Best Mode for Carrying out Invention

### Amphipathic, alpha-helical cell-penetrating peptide

The present disclosure provides a dimeric cell-penetrating peptide comprising, as a monomer, a 10-mer peptide that consists of a total of 10 amino acids. The 10-mer monomeric peptide may comprise hydrophobic amino acids and hydrophilic amino acids in an appropriate ratio, such as 1: 1 (more specifically, 4 each for the hydrophobic amino acid and the hydrophilic amino acid), and each monomer is linked to each other via a bond at one or two positions to form a dimeric peptide.

The present inventors intended to provide an optimal cell-penetrating peptide in consideration of dimer formation rate, cell-penetrating ability and cytotoxicity, correlation between nanoparticle self-association characteristic and cell-penetrating ability, and the like. Specifically, it was identified that the most optimal monomeric peptide was a monomeric peptide consisting of 10 amino acids in terms of dimer formation rate, and it was found that such a monomeric peptide had the optimal cell-penetrating ability in a case of being made into a dimer bundle. In particular, the dimeric peptide obtained using 10 amino acids has excellent cell-penetrating ability at a nanomolar level, and has low cytotoxicity as compared with a conventionally known dimeric peptide obtained using 16 amino acids, which makes the dimeric peptide advantageous for use *in vivo.* In addition, the short 10-mer cell-penetrating peptide penetrates faster into cells as compared with the existing 16-mer peptide, which is advantageous in that it is possible to rapidly deliver a modulator into cells.

The present disclosure provides a dimeric peptide, comprising, as a monomer, a peptide represented by Formula 1.

<Formula 1> X₁X₂X₃X₄X₅X₆X₇X₈X₉X₁₀

In the formula,
X₁, X₄, X₅, and X₈ are each independently a hydrophobic amino acid,
X₃, X₆, X₇, and X₁₀ are each independently a hydrophilic amino acid,
X₂ and X₉ are each independently an amino acid that forms a bond so that each monomeric peptide may be linked to each other at at least one position of X₂ and X₉ to form a dimeric peptide, and
X₁ is N-terminus and X₁₀ is C-terminus.

The dimeric peptide may be an amphipathic, alpha-helical peptide in the form of a homodimer or a heterodimer. For example, the dimeric peptide may be used in the form of a heterodimer in a case where an additional link is provided using a fluorescent substance, biotin, or lipid.

As used herein, the term "peptide" is an amino acid polymer and may comprise, as constituent elements, natural amino acids as well as non-natural amino acids.

In an embodiment, types of the amino acids that constitute the peptide are not limited as long as they are capable of maintaining an alpha-helical structure while exhibiting amphipathy, and hydrophobic amino acids or hydrophilic amino acids, which are known to those skilled in the art, may be appropriately selected and used. In a previous study, the present inventors revealed that the peptide having an increased alpha-helical content was capable of exhibiting increased intracellular penetrability while exhibiting decreased toxicity due to removal of the alpha helix in a case of being present in cells (WO 2015/057009).

In an embodiment, the hydrophobic amino acid may be selected from the group consisting of leucine (L), isoleucine (I), phenylalanine (F), valine (V), norvaline (norV), tryptophan (W), pentylglycine (pg), neopentylglycine (Npg), and cyclohexylalanine (Cha). Accordingly, X₁, X₄, X₅, and X₈ each independently may be a hydrophobic amino acid selected from the group consisting of leucine (L), isoleucine (I), phenylalanine (F), valine (V), norvaline (norV), tryptophan (W), pentylglycine (pg), neopentylglycine (Npg), and cyclohexylalanine (Cha).

In another embodiment, the hydrophilic amino acid may be selected from the group consisting of lysine (K), arginine (R), homoarginine (hR), norarginine (norR), histidine (H), omithine (O), diaminobutanoic acid (Dab), and diaminopropanoic acid (Dap). Accordingly, X₃, X₆, X₇, and X₁₀ each independently may be a hydrophilic amino acid selected from the group consisting of lysine (K), arginine (R), homoarginine (hR), norarginine (norR), histidine (H), omithine (O), diaminobutanoic acid (Dab), and diaminopropanoic acid (Dap).

The dimeric peptide of the present disclosure is formed via bonding of monomeric peptides to each other, in which the bonding may be made at any one or both positions of X₂ and X₉. Accordingly, X₂ and X₉ each independently may be selected from any amino acid that forms a bond so that each monomeric peptide may be linked to each other at at least one position of X₂ and X₉ to form a dimeric peptide. For example, X₂ and X₉ are each independently an amino acid selected from the group consisting of cysteine (C), homocysteine (Hcy), penicillamine (Pen), selenocysteine (Sec, U), and leucine (L), provided that X₂ and X₉ may not be leucine (L) at the same time.

Here, the bond formed between the respective monomeric peptides may include any type of bond linking the peptides to exhibit desired properties according to the present disclosure, and may include, for example, a covalent bond. The covalent bond is not particularly limited as long as it is a type of covalent bond capable of increasing an alpha-helical content without inhibiting functions of the peptide, and may be, for example, at least one bond selected from the group consisting of a disulfide bond between cysteines, a diselenide bond, an ester bond, a maleimide bond, a thioester bond, a thioether bond, and a bond formed by a click reaction. More specifically, the covalent bond may be at least one selected from the group consisting of a disulfide bond between cysteine (C), homocysteine (Hcy), or penicillamine (Pen) residues, a diselenide bond between selenocysteine (See, U) residues, an ester bond, a maleimide bond formed by using a thiol functional group capable of participating in a reaction, a thioester bond, a thioether bond, and a bond formed by a click reaction in a case where triple bond- or azide group-containing non-natural amino acids, which are capable of causing a click reaction, are contained.

In a case where a disulfide bond is formed between two monomers of a dimeric peptide, when cysteine (C) in the monomer is used, four atoms (CSSC) exist between the two monomeric peptide backbones. Here, when homocysteine (Hcy) is used instead of cysteine (C), it is possible to adjust the distance between the two monomeric peptide backbones to a 5-atom bonding distance (CCSSC or CSSCC) or a 6-atom bonding distance (CCSSCC). In an embodiment, a dimer may be used in which one cysteine and one homocysteine are linked through oxidation. In addition, selenocysteine (Sec, U) or penicillamine (Pen) may be used instead of cysteine to form a diselenide bond, hybridization with cysteine or penicillamine, or a disulfide bond of ester.

For each dimeric peptide, respective monomeric peptides may be linked to each other in a parallel direction where both peptides maintain a N-terminal to C-terminal direction, or may be linked to each other in such a fashion that one of the two peptides is in a N-terminal to C-terminal direction and the other is in a C-terminal to N-terminal direction, that is, in an anti-parallel direction. In an embodiment, each monomeric peptide is linked to each other in an anti-parallel direction.

The dimeric peptide may be lipidated at the N-terminus with a fatty acid. Specifically, the dimeric peptide may have a C₆ to C₁₆ fatty acid bound to a position of X₁. Such fatty acid binding enables the dimeric peptide to have further increased cell-penetrating ability.

In a specific embodiment, the present disclosure provides a dimeric peptide, comprising, as a monomer, a peptide represented by Formula 1.

<Formula 1> X₁X₂X₃X₄X₅X₆X₇X₈X₉X₁₀

In the formula,
X₁, X₄, X₅, and X₈ are each independently leucine (L), isoleucine (I), phenylalanine (F), valine (V), neopentylglycine (Npg), or cyclohexylalanine (Cha),
X₃, X₆, X₇, and X₁₀ are each independently lysine (K), arginine (R), homoarginine (hR), norarginine (norR), histidine (H), omithine (O), diaminobutanoic acid (Dab), or diaminopropanoic acid (Dap), and
X₂ and X₉ are each independently cysteine (C), homocysteine (Hcy), penicillamine (Pen), selenocysteine (Sec, U) or leucine (L), provided that X₂ and X₉ are not leucine (L) at the same time.

In particular, X₁, X₄, X₅, and X₈, which are hydrophobic amino acids, each independently may be phenylalanine (F), isoleucine (I), leucine (I), or neopentylglycine (Npg). X₃, X₆, X₇, and X₁₀, which are hydrophilic amino acids, each independently may be arginine (R) or lysine (K).

However, a monomeric peptide, which constitutes the dimeric peptide and is represented by Formula 1 where X₁, X₄, X₅, and X₈ are each independently leucine (L), cyclohexylalanine (Cha), or phenylalanine (F); X₂ and X₉ are cysteine (C); and X₃, X₆, X₇, and X₁₀ are all arginine (R), may be excluded from the scope of the present disclosure. That is, in an embodiment, for Formula 1, in a case where X₂ and X₉ are cysteine (C); and X₃, X₆, X₇, and X₁₀ are all arginine (R), X₁, X₄, X₅, and X₈ are each independently not leucine (L), cyclohexylalanine (Cha), or phenylalanine (F).

The present disclosure also provides the monomeric peptide itself as mentioned above. In an embodiment, the monomeric peptide of the present disclosure is represented by Formula 1.

<Formula 1> X₁X₂X₃X₄X₅X₆X₇X₈X₉X₁₀

In the formula,
X₁ to X₁₀ are as defined above.

In another embodiment, the monomeric peptide of the present disclosure is a peptide represented by Formula 1 or a peptide comprising an amino acid sequence selected from the group consisting of SEQ ID NOs: 3 and 8 to 46. As used herein, the term "monomeric peptide" refers to a peptide that is used as a basis for preparation of a dimeric peptide, and may be used interchangeably with "unit peptide".

In a more specific embodiment, the dimeric peptide of the present disclosure may comprise a monomeric peptide comprising an amino acid sequence selected from the group consisting of SEQ ID NOs: 3 and 8 to 26. In another example, the dimeric peptide of the present disclosure may comprise a monomeric peptide having an amino acid sequence selected from the group consisting of SEQ ID NOs: 3 and 8 to 26. In yet another example, the dimeric peptide of the present disclosure may comprise a monomeric peptide consisting of an amino acid sequence selected from the group consisting of SEQ ID NOs: 3 and 8 to 26. Here, the monomeric peptide consists of 10 amino acids, and each monomeric peptide may be linked to each other, via a covalent bond as described above, at at least one position of X₂ and/or X₉ to form a dimeric peptide.

The dimeric peptide according to the present disclosure is capable of retaining cell-penetrating ability even at a low concentration in a nanomolar concentration range. Conventional cell-penetrating peptides required a concentration that is about micromolar or higher to deliver a biologically active substance into cells. However, it has been identified that the cell-penetrating dimeric peptide of the present disclosure is capable of retaining desired cell-penetrating ability even at a nanomolar concentration, and thus can be advantageously used for effective delivery of a biologically active substance.

As such, by using the cell-penetrating dimeric peptide at a very small concentration, in particular, at a concentration that is several tens of nanomolar or lower, it is possible to achieve a desired effect even in a case where a biologically active substance is also used at a very low concentration as compared with conventionally used concentrations. A low concentration of cell-penetrating peptide and a low concentration of biologically active substance may constitute a sufficient condition to minimize cytotoxicity.

One of the most important points to be considered when using a cell-penetrating peptide is a balance between cell-penetrating ability and toxicity. Cell-penetrating peptides themselves have cell-penetrating ability, and thus may cause toxicity once they enter cells. That is, cell-penetrating ability and toxicity may be correlated to each other. Therefore, it is important to develop a peptide having high cell-penetrating ability while also having minimized toxicity. The dimeric peptide according to the present disclosure exhibits cell-penetrating ability even at a concentration that is several nanomolar to several hundreds of nanomolar, and is characterized in that a ratio of concentrations at which cell-penetrating ability and cytotoxicity are observed is 1/10 or lower. In a case where a cell-penetrating peptide is used at a low concentration to deliver a bioactive substance into cells, cytotoxicity caused by the cell-penetrating peptide may be minimized.

In a case where the cell-penetrating dimeric peptide according to the present disclosure penetrates into the cytoplasm that is at a reducing condition, the dimeric peptide may undergo breaking of a covalent bond formed therebetween to become monomeric peptides. In a case where the covalent bond is maintained in cells and the peptide still exists in the form of a dimeric peptide, high cytotoxicity may occur because the dimeric peptide generally has excellent binding ability to DNA, RNA, or the like. However, a monomeric peptide resulting from breaking of a covalent bond in the cytoplasm has very low chemical stability due to a sharply decreased alpha-helical content, and thus may be easily hydrolyzed by many proteolytic enzymes in cells.

Accordingly, the cell-penetrating dimeric peptide according to the present disclosure is not only capable of exhibiting excellent intracellular delivery ability, but also capable of achieving a desired effect even in a case where a biologically active substance is used at a low concentration and capable of exhibiting minimized cytotoxicity by being decomposed in cells.

In a specific embodiment, a ratio of cell-penetrating ability to cytotoxicity (IC₅₀⁻¹) at 62.5 nM for the dimeric peptide of the present disclosure is higher than a conventionally known peptide (for example, a dimer of LK-3). For example, the ratio may be 130,000 or higher.

In another specific embodiment, a ratio of nanoparticle self-association characteristic (ΔΔRt) to cytotoxicity (IC₅₀⁻¹) at 62.5 nM for the dimeric peptide of the present disclosure is higher than a conventionally known peptide (for example, a dimer of LK-3). For example, the ratio may be 8 or higher.

### Composition for intracellular delivery

The present disclosure provides a platform technology capable of delivering a biologically active substance, such as a therapeutic drug, into cells using the cell-penetrating peptide.

The dimeric peptide according to the present disclosure exhibits cell-penetrating ability. Thus, use of this peptide makes it possible to effectively use a biologically active substance that has not been able to exhibit effective therapeutic efficacy and the like due to conventional difficulties in delivery thereof into cells.

Accordingly, the present disclosure provides a pharmaceutical composition comprising the dimeric peptide and a biologically active substance.

The biologically active substance is a type of cargo and may be a substance that is bound to a cell membrane-penetrating domain and delivered into cells to regulate biological activity or function which regulates all physiological phenomena in a living body. For example, the biologically active substance may be, but is not limited to, DNA, RNA, siRNA, an aptamer, a protein, an antibody, a small molecule compound, or a cytotoxic compound.

To the dimeric peptide according to the present disclosure may additionally be bound a substance or other carrier that modulates biological activity or function, in which the dimeric peptide and the substance or other carrier that modulates biological activity or function may form a complex structure. The substance or carrier may be linked to the peptide of the present disclosure via, for example, a non-covalent or covalent bond formed therebetween. The non-covalent bond may be, for example, at least one selected from the group consisting of hydrogen bond, electrostatic interaction, hydrophobic interaction, Van der Waals interaction, pi-pi interaction, and cation-pi interaction. The covalent bond may be a degradable or non-degradable bond. The degradable bond may be a disulfide bond, an acid-degradable bond, an ester bond, an anhydride bond, a biodegradable bond, or an enzymatically degradable bond, and the non-degradable bond may be an amide bond or a phosphate bond; however, the degradable bond and the non-degradable bond are not limited thereto.

In a case where the biologically active substance is a cytotoxic compound, the cytotoxic compound may be linked to the dimeric peptide by forming a non-covalent bond, such as electrostatic bond or host-guest bond, therewith. The cytotoxic compound may be, but is not limited to, doxorubicin, methotrexate, paclitaxel, cisplatin, bleomycin, taxol, berberine, or curcumin. In a case where the biologically active substance is a protein or antibody, the protein or antibody may include any type of drug that specifically binds to a specific target in cells, and may be introduced into the peptide in the form of being fused to the N-terminus or C-terminus thereof.

In some cases, for drug-resistant cancer cells (for example, MCF7 or MDA-MB-231), the dimeric peptide to which methotrexate (MTX) is bound may be used as a new substance capable of killing the cancer cells, and may have an increased intracellular delivery concentration in a case where a bioactive small molecule (taxol, berberine, curcumin, or the like) with hydrophobicity is attached thereto. In a specific example, in a case where MTX is covalently bound to the dimeric peptide according to the present disclosure, drug efficacy of MTX was increased 20-fold or higher in MTX-resistant MDA-MB-231 cells. In some cases, it is possible to provide a cell-penetrating peptide that acts at a concentration as much as 40-fold lower.

In addition, an antibody, a protein that is a part of an antibody, or a protein drug may be linked to the dimeric peptide and delivered into cells; and an oligonucleotide drug (siRNA, asDNA, DNA, or aptamer) may be linked to the dimeric peptide and delivered into cells. Alternatively, a biologically active substance (for example, an antibody) may be linked to the cell-penetrating peptide via a non-covalent bond, such as biotin-streptavidin, and delivered into cells.

The composition according to the present disclosure may further comprise one or more pharmaceutically acceptable carriers. The pharmaceutically acceptable carrier should be compatible with the active ingredient of the present disclosure, and may be one selected from physiological saline, sterile water, Ringer's solution, buffered saline, dextrose solution, maltodextrin solution, glycerol, ethanol, and a mixture of two or more thereof. In addition, a diluent, a dispersing agent, a surfactant, a binder, and a lubricant may be additionally added to the composition to prepare an injectable formulation such as aqueous solution, suspension, and emulsion. In particular, it is preferable to provide the formulation in a lyophilized form. For the preparation of a lyophilized formulation, a method commonly known in the art to which the present disclosure pertains may be used, and a stabilizer for lyophilization may be added. Furthermore, the composition may preferably be formulated with an appropriate method known in the art depending on diseases or ingredients.

Hereinafter, the present disclosure will be described in more detail by way of examples. These examples are only for illustrating the present disclosure, and it will be apparent to those of ordinary skill in the art that the scope of the present disclosure is not to be construed as being limited by these examples.

### Mode for Carrying out Invention

### Example 1. Synthesis of monomeric and dimeric peptides, and comparison of dimer formation rates

Each of the monomeric peptides in Table 1 was prepared and subjected to an oxidation condition in air to produce each dimer.

**[Table 1]**

| SEQ ID NO. | Monomer (dimer) | Amino acid sequence of monomer |
|---|---|---|
| 1 | LK-2 (LK-3) | Ac-LKKLCKLLKKLCKLAG-NH₂ |
| 2 | LK8 (diLK8) | Ac-CKLLKKLC-NH₂ |
| 3 | LK10 (diLK10) | Ac-LCKLLKKLCK-NH₂ |
| 4 | LK12 (diLK12) | Ac-KLCKLLKKLCKL-NH₂ |
| 5 | LK14 (diLK14) | Ac-KKLCKLLKKLCKLL-NH₂ |
| 6 | LK16 (diLK16) | Ac-LKKLCKLLKKLCKLLK-NH₂ |
| 7 | LK18 (diLK18) | Ac-LLKKLCKLLKKLCKLLKK-NH₂ |

First, each monomeric peptide was synthesized using a solid-phase peptide synthesis method. Specifically, the synthesis was carried out using a standard fluorenylmethyloxy carbonyl (Fmoc) solid-phase peptide in a peptide microwave synthesizer (CEM). Rink amide MBHA resin (0.59 mmole/g loading, 50 mg, 29.5 µmol) was used in Discover SPS. The resin was deprotected with 20% piperidine in DMF. Coupling reaction was performed using amino acids of each sequence, PyBOP, and N-diisopropylethylamine (DIPEA). Fluorescence labeling was performed by allowing 5-TAMRA to react at the N-terminus of the synthesized peptide in the presence of HOBt, HCTU, and DIPEA. The synthesized peptide was isolated from the resin using a cleavage cocktail for 2 hours at room temperature (in which 940 µL of trifluoroacetic acid, 25 µL of 1,2-ethanedithiol, 25 µL of water, and 10 µL of triisopropylsilane were used). The isolated peptide was precipitated with n-hexane and diethyl ether (v/v = 1/1) and purified by reverse phase chromatography HPLC. Purification was performed using HPLC (Agilent HPLC 1100 series) with a Zorbax C18 column (3.5 µm, 4.6×150 mm).

HPLC conditions: buffer A (water with 0.1 % v/v TFA) and buffer B (acetonitrile with 0.1 % v/v TFA), flow rate of 1 mL/min; 5 min, 5% B, followed by a linear gradient of 70% B over 25 minutes to 100% B over 10 minutes, followed by hold for 10 minutes.

The prepared monomer was subjected to an oxidation condition in air (air oxidation in 0.1 M NH₄HCO₃ aqueous solution) to produce a dimer.

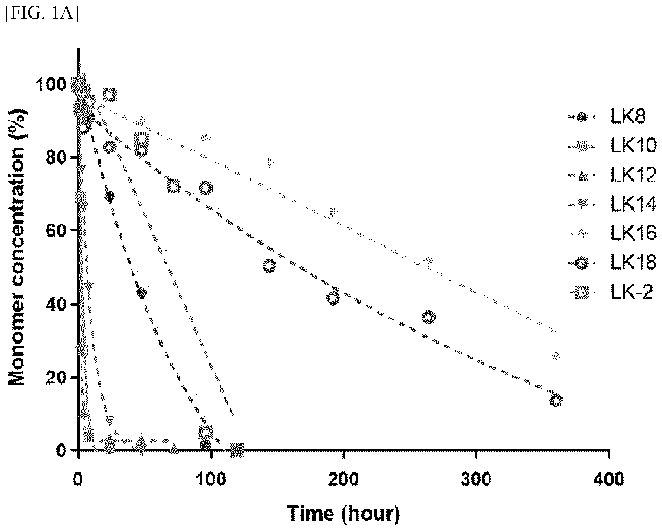

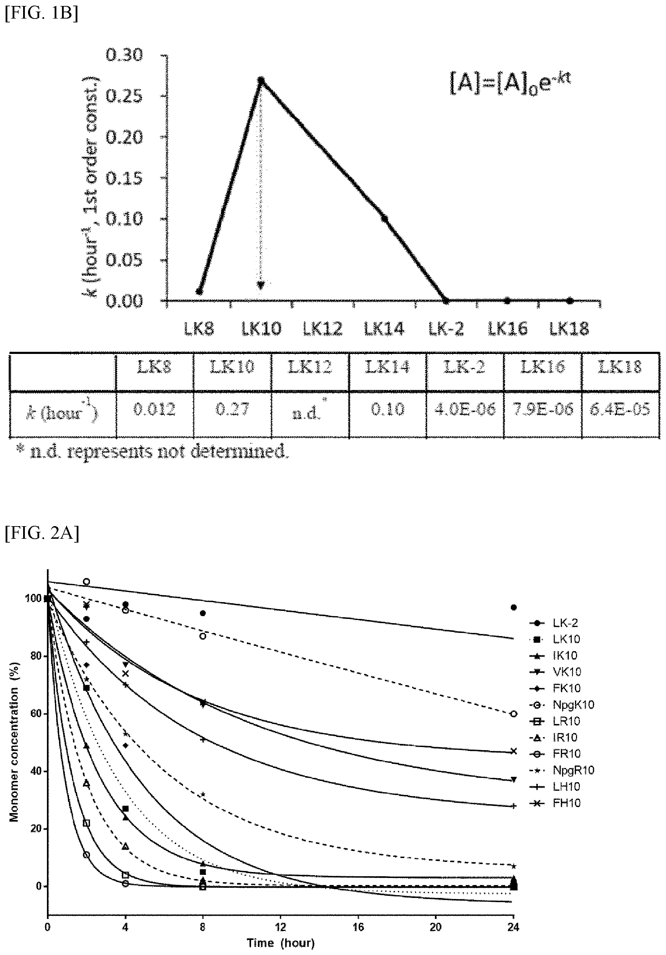

Here, it was assumed that a dimer production rate and a monomer disappearance rate were the same. Thus, the dimer production rate was expressed as the inverse of the monomer disappearance rate. Each experiment was carried out at a condition where the monomer was present at the same concentration (the monomer at a concentration of 1 mM was subjected to air oxidation in 0.1 M NH₄HCO₃), and the results are illustrated in FIG. 1.

Surprisingly, a dimer was produced most rapidly from a monomeric peptide (LK10) consisting of 10 amino acids, in which 90% or higher of the reaction was completed within 8 hours, and almost 100% of the reaction was completed within 24 hours. On the other hand, in a case where a dimer was produced from a monomeric peptide (LK16 or LK18) consisting of 16 or 18 amino acids, it took several days for 100% reaction to proceed. It was observed that in a case where a dimer was produced from a monomeric peptide (LK14) consisting of 14 amino acids, the reaction proceeded relatively fast but was slower than LK10, and that even in a case where a dimer was produced from a shorter monomeric peptide (LK8) consisting of 8 amino acids, the reaction proceeded more slowly than LK10. It was observed that in a case where a dimer was produced from a monomeric peptide (LK12) consisting of 12 amino acids, the reaction proceeded the fastest but crystal formation occurred rapidly, in which the monomers produced various types of oligopeptides (trimers, tetramers, and the like) rather than forming dimers. LK10 exhibited a fast dimer production rate even as compared with the conventionally known monomeric peptide (LK-2) consisting of 16 amino acids.

Even in a case where actual first-order reaction rate constant k values are compared, it was identified that LK10 exhibited the highest value (FIG. 1B).

From a commercial point of view and the like, it is important that CPP be able to rapidly form an amphipathic dimer. Thus, a peptide consisting of 10 amino acids was identified as having an optimized length in terms of a dimer production rate (formation rate).

### Example 2. Synthesis of 10-mer monomeric and dimeric peptides, and comparison of dimer formation rates

Each of the monomeric peptides in Table 2 was prepared in the same manner as in Example 1, and subjected to an oxidation condition to produce each dimer.

**[Table 2]**

| SEQ ID NO | Monomer (dimer) | Amino acid sequence of monomer |
|---|---|---|
| 8 | FK10 (diFK10) | Ac-FCKFFKKFCK-NH₂ |
| 9 | FR10 (diFR10) | Ac-FCRFFRRFCR-NH₂ |
| 10 | IR10 (diIR10) | Ac-ICRIIRRICR-NH₂ |
| 11 | LR10 (diLR10) | Ac-LCRLLRRLCR-NH₂ |
| 12 | IK 10 (diIK10) | Ac-ICKIIKKICK-NH₂ |
| 13 | L*R10 (di L*R10) | Ac-L*CRL*L*RRL*CR-NH₂(L* = Npg) |
| 14 | LH10 (diLH10) | Ac-LCHLLHHLCH-NH₂ |
| 15 | VK10 (diVK10) | Ac-VCKVVKKVCK-NH₂ |
| 16 | FH10 (diFH10) | Ac-FCHFFHHFCH-NH₂ |
| 17 | L*K10 (di L*K10) | Ac-L*CKL*L*KKL*CK-NH₂ (L* = Npg) |
| 18 | VH10 (diVH10) | Ac-VCHVVHHVCH-NH₂ |
| 19 | L*H10 (di L*H10) | Ac-L*CHL*L*HHL*CH-NH₂ (L* = Npg) |
| 20 | VR10 (diVR10) | Ac-VCRVVRRVCR-NH₂ |
| 21 | IH10 (diIH10) | Ac-ICHIIHHICH-NH₂ |
| 22 | L'K10 (diL'K10) | Ac-L'CKL'L'KKL'CK-NH₂ (L' = Cha) |
| 23 | LR10^{C2L} (diLR10^{C2L}) | Ac-LLRLLRRLCR-NH₂ |
| 24 | LR10^{C9L} (diLR10^{C9L}) | Ac-LCRLLRRLLR-NH₂ |
| 25 | LO10 (diLO10) | Ac-LCOLLOOLCO-NH₂ (O = Orn) |
| 26 | LK'10 (diLK'IO) | Ac-LCK'LLK'K'LCK'-NH₂ (K' = Dab) |
| 27 | LK"10 (diLK"10) | Ac-LCK"LLK"K"LCK"-NH₂ (K" = Dap) |
| 28 | LhR10 (diLhR10) | Ac-LChRLLhRhRLChR-NH₂ |
| 29 | LR^{∗}10 (diLR^{∗}10) | Ac-LCR*LLR*R*LCR*-NH₂ (R* = norR) |
| 30 | LR10^{R3K} (diLR10^{R3K}) | Ac-LCKLLRRLCR-NH₂ |
| 31 | LR10^{R6K} (diLR10^{R6K}) | Ac-LCRLLKRLCR-NH₂ |
| 32 | LR10^{R7K} (diLR10^{R7K}) | Ac-LCRLLRKLCR-NH₂ |
| 33 | LR10^{R10K} (diLR10^{R10K}) | Ac-LCRLLRRLCK-NH₂ |
| 34 | LR10^{R7,10K} (diLR10^{R7,10K}) | Ac-LCRLLRKLCK-NH₂ |
| 35 | LR10^{R6,10K} (diLR10^{R6,10K}) | Ac-LCRLLKRLCK-NH₂ |
| 36 | LR10^{R6,7K} (diLR10^{R6,7K}) | Ac-LCRLLKKLCR-NH₂ |
| 37 | LR10^{R3,10K} (diLR10^{R3,10K}) | Ac-LCKLLRRLCK-NH₂ |
| 38 | LR10^{R3,7K} (diLR10^{R3,7K}) | Ac-LCKLLRKLCR-NH₂ |
| 39 | LR10^{R3,6K} (diLR10^{R3,6K}) | Ac-LCKLLKRLCR-NH₂ |

Among these, dimer formation rates of the monomeric peptides of SEQ ID NOs: 8 to 17 were measured and compared with LK10. The results are illustrated in FIG. 2A. Formation of dimers rapidly occurred for the 10-mer monomers. In particular, FK10, LK10, FR10, IR10, LR10, and IK10 had a fast dimer formation rate that was almost similar to LK10, in which almost 100% of the reaction was completed within 24 hours, and NpgR10 also had a particularly fast dimer formation rate (FIG. 2A).

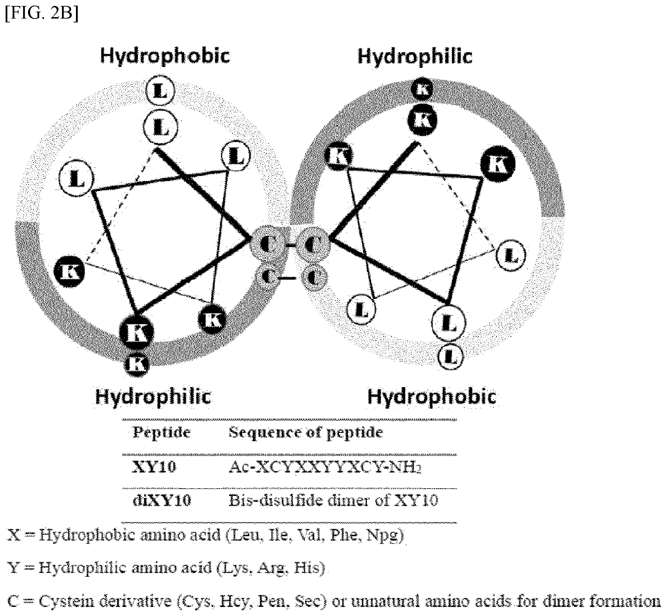

From the results, it was identified that for a monomer consisting of 10 amino acids, in particular, in which Leu, Ile, Val, Phe, or Npg is located on the hydrophilic surface, and Lys, Arg, or His is located on the hydrophilic surface, formation of a dimeric peptide proceeded rapidly. The peptide that satisfies this condition is illustrated as a helical wheel image in FIG. 2B.

### Example 3. Identification of changes in dimer formation ability depending on changes in amino acid sequence of monomer

The peptide of the present disclosure is an amphipathic, alpha-helical peptide in which a hydrophilic residue region and a hydrophobic residue region of the peptide are distinguished when forming the alpha-helical form. In order to identify an effect of this amphipathy on dimer-forming ability, the amino acid sequence of a monomer was partially changed to decrease amphipathy, and it was checked whether this monomer formed a dimer.

For this purpose, starting from the sequence of LR10 (Ac-LCRLLRRLCR-NH₂), position exchange was performed between leucine at position 8, which is a hydrophobic surface, and arginine at position 10, which is a hydrophilic surface, to prepare the monomer LR10^{8,10exch} (Ac-LCRLLRRRCL-NH₂) having decreased amphipathy. Dimer formation ability of the monomer was identified under the same oxidizing condition. As a result, a dimer of LR10^{8,10exch} was not formed, and kink formation occurred mainly due to a disulfide bond formed between cysteine at position 2 and cysteine at position 9 in the monomer. These results suggest that amphipathy in an alpha-helical structure of the peptide has a major influence on dimer formation.

In addition, RL10 (Ac-RCLRRLLRCL-NH₂) was prepared by changing directionality while maintaining amphipathy starting from the peptide, and dimer formation ability thereof was identified. For RL10, some kink formation occurred under an oxidizing condition, similarly to LR10^{8,10exch}, and at the same time, formation of insoluble white precipitates also occurred. From these results, it can be seen that not only amphipathy of the peptide, but also directionality thereof has a great influence on dimer formation ability.

Accordingly, it can be seen that in order for a monomer of an amphipathic, alpha-helical peptide of the present disclosure to efficiently form a dimer, amphipathy and directionality of the monomer have to be maintained.

### Example 4. Preparation of monomeric and dimeric peptides with varying spacing between dimeric bundle peptides

Two disulfide bonds are linked between monomeric peptides to form a dimer. In a case where a monomeric peptide was produced by replacing cysteine (Cys), which forms a disulfide bond, with homocysteine (Hcy) having one more carbon atom in a chain, and a dimeric peptide was produced using this monomeric peptide, it was possible to obtain a new dimer with increased spacing between the two monomeric peptides. For example, it was possible to obtain LK-3+1 by mixing LK-2 (SEQ ID NO: 1) and LK-2^{C5Hcy/C12Hcy} (SEQ ID NO:48) in a 1:1 molar ratio, subjecting the mixture to an oxidation condition in air to obtain dimers in heterodimeric form, and isolating the dimers.

The peptides in Table 3 were prepared according to the same method as in Example 1.

**[Table 3]**

| **SEQ ID NO** | **Monomer (dimer)** | **Amino acid sequence of monomer** |
|---|---|---|
| 40 | LK10^{C9Hcy} (diLK10^{C9Hcy}) | Ac-L**C**KLLKKL**Hcy**K-NH₂ |
| 41 | LK10^{C2Hcy/C9Hcy} (diLK10^{C2Hcy/C9Hcy}) | Ac-L**Hcy**KLLKKL**Hcy**K-NH₂ |
| 42 | LR10^{C2Hcy} (diLR10^{C2Hcy}) | Ac-L**Hcy**RLLRRL**C**R-NH₂ |
| 43 | LR10^{C9Hcy} (diLR10^{C9Hcy}) | Ac-L**C**RLLRRL**Hcy**R-NH₂ |
| 44 | LR10^{C2Hcy/C9Hcy}, (diLR10^{C2Hcy/C9Hcy}) | Ac-L**Hcy**RLLRRL**Hcy**R-NH₂ |
| 45 | LK10^{C2U/C9U}, (diLK10^{C2U/C9U}) | Ac-LUKLLKKLLUK-NH₂ |
| 46 | LK10^{C2Pen/C9Pen}, (diLK10^{C2Pen/C9Pen}) | Ac-L**Pen**KLLKKL**Pen**K-NH₂ |
| 47 | LK-2^{C12Ecy} (LK-3^{C12Hcy}) | Ac-LKKLCKLLKKLHcyKLAG-NH₂ |
| 48 | LK-2^{C5Hcy/C12Hcy} (LK-3^{C5Hcy/C12Hcy}) | Ac-LKKLHcyKLLKKLHcyKLAG-NH₂ |

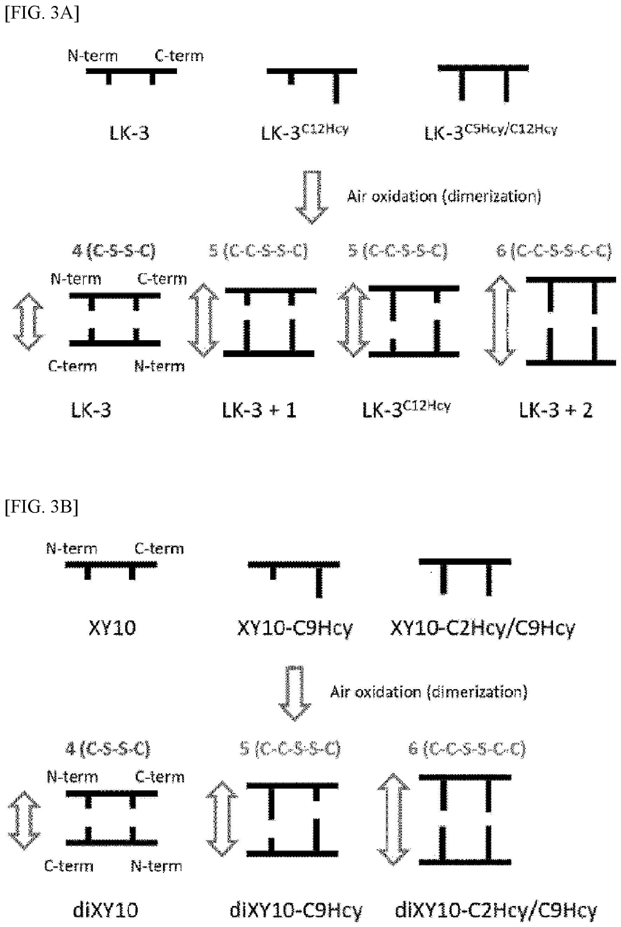

For better understanding, processes of adjusting spacing between monomers are schematically illustrated in FIG. 3. Depending on the homocysteine (Hcy) level used, the distance between the monomeric peptides was divided into 4 atoms (CSSC; where only Cys is used), 5 atoms (CCSSC or CSSCC), or 6 atoms (CCSSCC).

### Example 5. Identification of cell-penetrating ability of dimeric peptides

During preparation of CPPs in dimeric form shown in Tables 1 to 3, one monomer thereof was prepared in a form where 5-TAMRA fluorescence was attached to the N-terminus, instead of acetyl. A monomer with acetyl and a monomer with 5-TAMRA fluorescence were mixed in a molar ratio of 1:1, and subjected to an oxidation condition in air to produce a dimeric form in which only one fluorescence was labeled per dimer molecule. Then, cell-penetrating ability of each dimer was identified. Each fluorescently labeled peptide was used to treat HeLa cells (5 × 10⁴ cells/well), which are human-derived cells, and a ratio of the number of cells with fluorescence to the total number of cells was obtained through flow cytometry.

The results obtained by identifying cell-penetrating ability depending on concentrations of respective dimeric peptides are illustrated in FIG. 4.

The dimeric peptide according to the present disclosure exhibited excellent cell-penetrating ability at the several tens of nanomolar level (FIG. 4A). FIGS. 4B to 4D illustrate results obtained by treating cells with respective dimers at the same concentration (62.5 nM), measuring fluorescence intensity of the cells by flow cytometry, and relatively comparing the fluorescence intensity.

FIG. 4E illustrates results obtained by identifying cell-penetrating ability of dimeric peptides depending on modification of spacing therein. For diLR10, it was possible to obtain the best results in a case of using diLR10^{C9Hcy} that has an increased length (that is, length of 5 atoms) between the monomers due to one carbon atom. For diLK10, similar results were obtained. For diL*R10 (that is, diNpgR10), similarly excellent results were obtained in a case of using dimeric peptides whose spacing between monomors was a length of 4 or 5 atoms. From these results, it was identified that the best cell-penetrating ability was obtained in a case where spacing between two monomers, which form a dimer, was a length of 5 atoms.

### Example 6. Identification of cytotoxicity of dimeric peptides

Cytotoxicity was identified using WST-1 assay by treating HeLa cells with peptides in a 96-well plate (1 × 10⁴ cells/well) for 24 hours.

Specifically, HeLa cells were cultured in DMEM at a condition of 37°C with 5% CO₂. The cells were respectively cultured in a cell culture plate, and then removed with trypsin. The cells were seeded at 1×10⁴ cells per well in a 96-well plate. After 24 hours, the peptide was added to the media, and culture was performed again at a condition of 37°C with 5% CO₂ for 24 hours. 10 uL of WST-1 reagent was added per well, and reaction was allowed to proceed for 30 minutes at a condition of 37°C with 5% CO₂. Then, UV absorbance was measured at 450/700 nm with a 96-well plate reader.

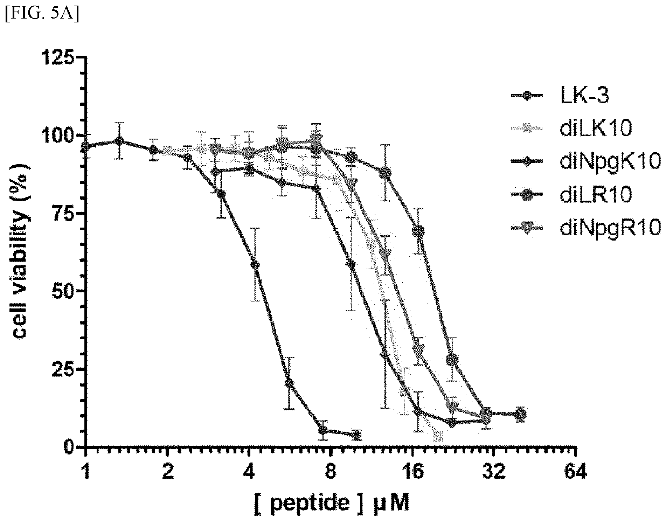

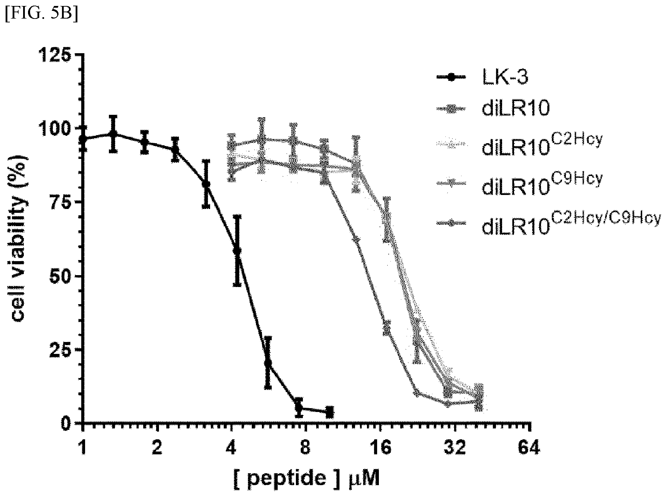

The results are shown in Table 4 and FIG. 5.

It was identified that as compared with LK-3, which was an existing dimeric peptide of 16-mer CPP, dimeric peptides formed of 10-mer CPPs had significantly lower cytotoxicity even at a high concentration. It was identified that even in a case of comparing IC50 values, the 10-mer CPPs had 2-fold or higher IC50 than LK-3, and thus had decreased toxicity to that extent (see ratio in Table 4). Even for diLH10, no toxicity was observed even at 40 µM (data not shown). In particular, it was identified that cytotoxicity of diLR10 decreased the most, which corresponded to an about 5-fold increase in IC50 value as compared with the existing 16-mer dimer LK-3. It was identified that diLR10 had an IC50 value of about 20 µM.

**[Table 4]**

| | LK-3 | diLK 10 | diIK 10 | diFK 10 | diL*K10 | diLR10 | diIR10 | diFR10 | diL*R10 |
|---|---|---|---|---|---|---|---|---|---|
| IC₅₀ (µM) | 4.44 | 12.4 | 11.4 | 9.36 | 10.3 | 18.9 | 14.4 | 18.2 | 13.8 |
| ratio | 1.00 | 2.79 | 2.57 | 2.11 | 2.32 | 4.26 | 3.24 | 4.10 | 3.11 |

It was identified whether cytotoxicity changed depending on spacing in the dimers. Low cytotoxicity was observed for a case where the spacing was a length of 5 atoms (CCSSC) (FIG. 5B).

### Example 7. Correlation between cytotoxicity and cell-penetrating ability/nanoparticle self-association characteristic of dimeric peptides

### 7.1 Correlation between cytotoxicity and cell-penetrating ability of dimeric peptides

Based on cell-penetrating ability and cytotoxicity of the dimeric peptides of the present disclosure, correlation therebetween was identified. Cytotoxicity (IC₅₀⁻¹) and cell-penetrating ability of the respective peptides were identified at 62.5 nM according to the methods described in Examples 5 and 6, and then graphically represented.

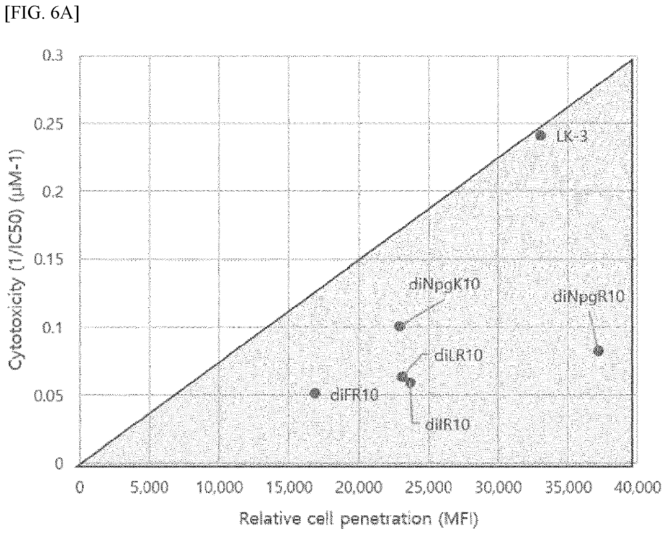

The results are illustrated in FIG. 6A.

As compared with LK-3, which was an existing dimeric peptide of 16-mer CPP, the dimeric peptides diNpgK10, diLR10, diFR10, diIR10, and diNpgR10 of the present disclosure had low toxicity as compared with cell-penetrating ability. That is, LK-3 had a ratio of cell-penetrating ability to cytotoxicity (IC₅₀⁻¹)(that is, cell-penetrating ability/cytotoxicity IC₅₀⁻¹) of about 130,000; however, all of the dimeric peptides of the present disclosure showed a higher ratio than LK-3. This indicates that the dimeric peptides of the present disclosure have an appropriate balance between cell-penetrating ability and cytotoxicity, and thus are suitable for use as a cell-penetrating peptide as compared with the existing dimeric peptide LK-3.

### 7.2 Correlation between cytotoxicity and nanoparticle self-association characteristic of dimeric peptides

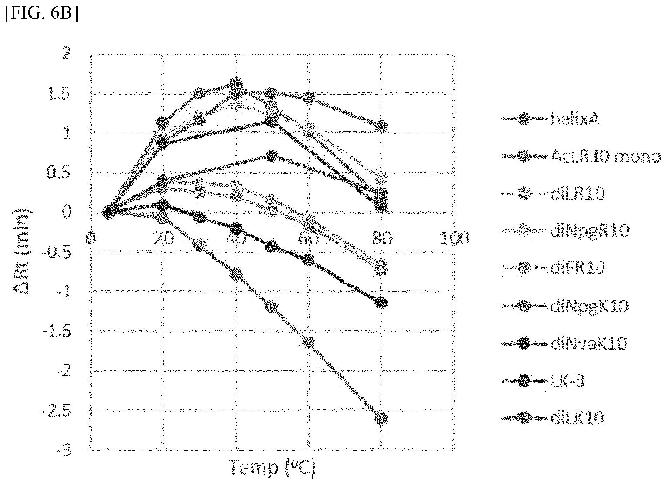

First, in order to identify whether the dimeric peptides of the present disclosure exhibit a self-association characteristic due to interaction between the peptides, an experiment capable of quantifying the self-association characteristic (Ref. JBC (2003) 278, 22918) was conducted. This experiment was conducted by observing retention time (Rt) in a C18 reversed-phase HPLC column depending on changes in temperature, and self-association tendency was determined by changes in the retention time. The results are illustrated in FIG 6B. Then, it was identified through a separate experiment that the dimeric peptides had stronger cell-penetrating ability as they had a stronger self-association characteristic (results not shown).

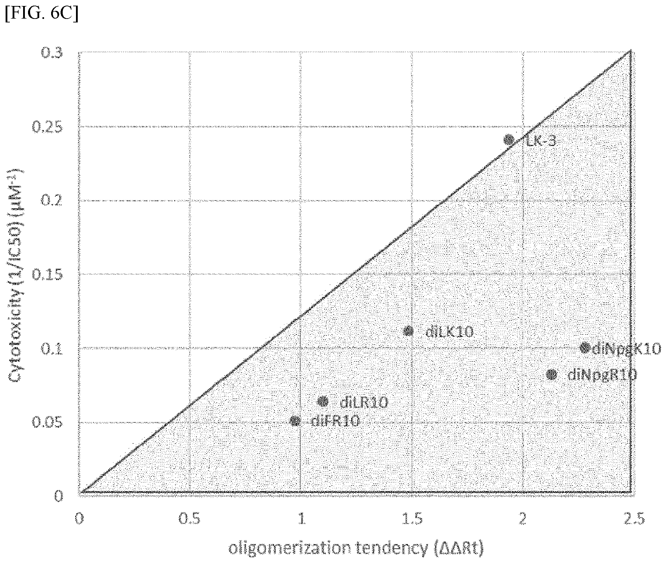

In addition, correlation between self-association tendency and cytotoxicity for each dimeric peptide was identified. Cytotoxicity (IC₅₀⁻¹) of each peptide was identified at 62.5 nM according to the method described in Example 6. Then, a ratio of nanoparticle self-association characteristic (ΔΔRt) to cytotoxicity (IC₅₀⁻¹) at 62.5 nM (that is, nanoparticle self-association characteristic (ΔΔRt)/cytotoxicity IC₅₀⁻¹) was identified. The results are illustrated in FIG. 6C.

It was found that for each dimeric peptide, self-association tendency was correlated with cytotoxicity. In particular, it was identified that as compared with LK-3, which was an existing dimeric peptide of 16-mer CPP, the dimeric peptides diFR10, di-LR10, diLK10, diNpgR10, and diNpgK10 of the present disclosure showed some low cytotoxicity as compared with self-association tendency. That is, LK-3 had a ratio of nanoparticle self-association characteristic to cytotoxicity (IC₅₀⁻¹) of about 8; however, all of the dimeric peptides of the present disclosure showed a higher ratio than LK-3.

The results in FIGS. 6A and 6C suggest that cell-penetrating ability of the dimeric peptides of the present disclosure is determined by their nanoparticle self-association characteristic. In particular, the results indicate that the dimeric peptides of the present disclosure have relatively low cytotoxicity and thus are optimized for *in vivo* use.

### Example 8. Preparation of parallel or antiparallel dimeric peptide and identification of cell-penetrating ability thereof

As shown in Table 5, the parallel or antiparallel dimer was prepared using the amino acid cysteine having different protecting groups.

**[Table 5]**

| | |
|---|---|
| Parallel | 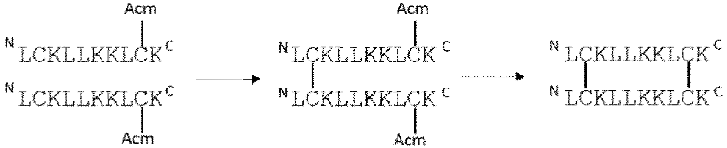 |
| Antiparallel | 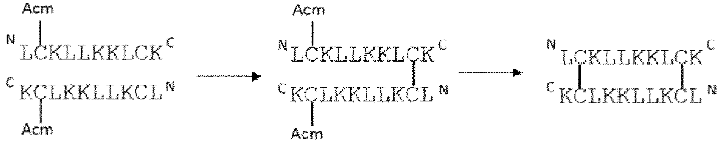 |

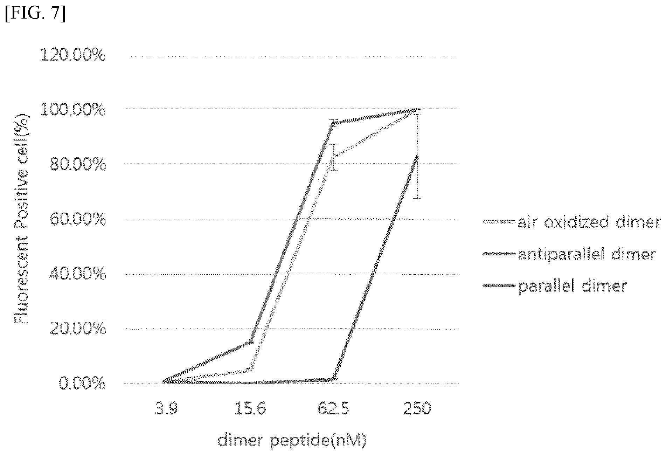

The results obtained by identifying cell-penetrating ability of the dimers are illustrated in FIG. 7.

The air oxidized dimer tended to exhibit cell-penetrating ability that was similar to the antiparallel dimer and was completely different from the parallel dimer, indicating that diLK10, which was naturally produced under an oxidation condition in air, had antiparallel alignment.

### Example 9. Identification of drug delivery effect of methotrexate-bound peptide (drug delivery by covalent bond)

In order to identify a drug delivery effect of a peptide, a peptide in which methotrexate (MTX) is bound to the N-terminus of one monomer of a dimer was synthesized according to a known method (Kim et al. Biomacromolecules 2016).

Table 6 shows IC50 values of MTX in three cell lines. A high IC50 value was observed in MDA-MB-231 cells, suggesting that these cells were resistant to MTX.

**[Table 6]**

| Cell line | NIH-3T3 | HeLa | MDA-MB-231 |
|---|---|---|---|
| IC₅₀ of MTX (µM) | 0.037 | ∼0.031 | 0.97 |

Accordingly, cytotoxicity was identified in MDA-MB-231 cells exhibiting resistance to MTX using the WST-1 assay described in Example 5. MDA-MB-231 cells were seeded in a 96-well plate at a density of 2.5 × 10³ cells/well, and treated with the MTX-bound peptide for 48 hours starting from the next day. The results are shown in Table 7.

**[Table 7]**

| | | | | | |
|---|---|---|---|---|---|
| IC₅₀ (µM) | MTX | diLK 10 | diLR10 | diNpgK 10 | diNpgR 10 |
| IC₅₀ of MTX-bound peptide | 0.972 | 0.0459 | 0.0401 | 0.0246 | 0.0316 |
| IC₅₀ | - | ∼13.7 | ∼8.92 | ∼5.84 | 11.2 |

As shown in Table 7, the MTX-bound peptide had an IC₅₀ value at the several tens of nanomolar level. From the viewpoint that a peptide itself has an IC₅₀ value at the several micromolar level, it was identified that the peptide according to the present disclosure was able to efficiently deliver MTX into cells with low toxicity at a nanomolar level.

### Example 10. Identification of drug delivery effect of complex of cyclosporine and peptide (drug delivery by non-covalent bond)

It was intended to identify whether a complex formed by simply mixing a peptide and a drug had an effect of delivering the drug.

The hydrophobic drug cyclosporine (CsA) was mixed with a peptide and incubated at room temperature for 20 minutes to create a complex with the hydrophobic drug. Then, Jurkat cells (1.0 × 10⁴ cells/well) were treated with the complex for 4.5 hours. An amount of extracellularly secreted IL-2 was measured by an ELISA method to identify, through IC50 values, whether an anti-inflammatory effect caused by CsA, which corresponded to a relative decrease in IL-2, was enhanced by the cell-penetrating peptide diLR10.

The results are shown in Table 8. From these results, it was identified that the IC50 value decreased by 21% in a case where diLR10 was used.

**[Table 8]**

| | CsA only | Complex w/ diLR10 |
|---|---|---|
| IC₅₀ (nM) | 14 | 11 |

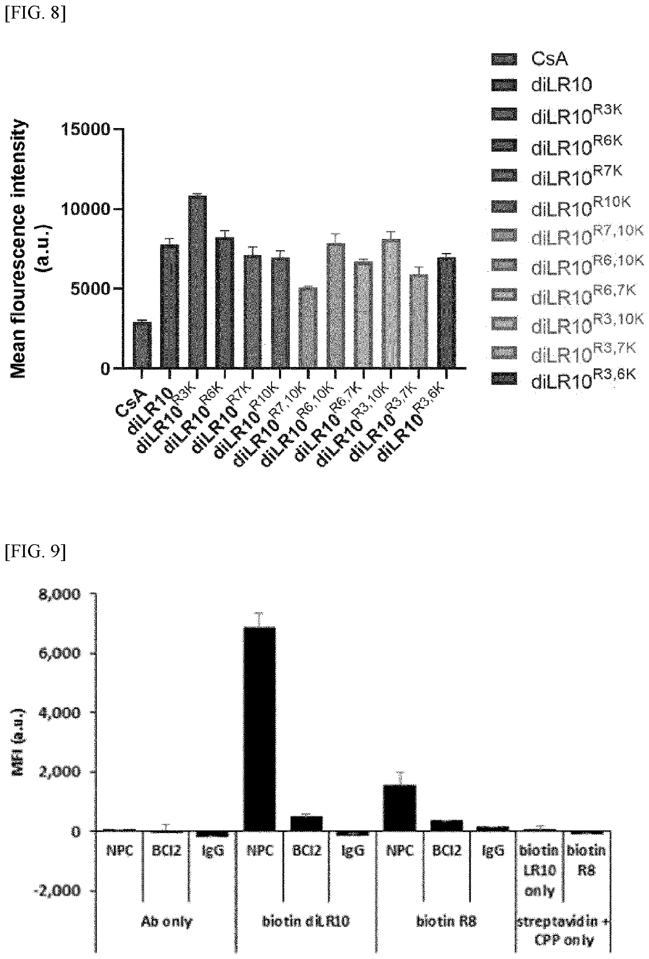

For the peptides with various compositions of lysine and arginine, their intracellular delivery by non-covalent bonding with CsA was identified using CsA with fluorescence. The results are illustrated in FIG. 8, in which diLR10^{R3K} was identified as having the best intracellular delivery ability for CsA.

### Example 11. Identification of drug (siRNA and antibody) delivery effect using streptavidin (drug delivery by pseudo-covalent bond)

In addition to the possibility of intracellular delivery of a hydrophobic drug by a non-covalent bond with the dimeric cell-penetrating peptide, it was identified whether it was possible to achieve drug delivery by a pseudo-covalent bond. That is, for an intracellular delivery method using streptavidin and biotin which do not form a covalent bond but have a binding affinity (Kd = 10⁻¹⁵ M) similar to a covalent bond, it was attempted to determine whether it was possible to deliver siRNA or antibody, which is a drug having difficulty in penetrating cells, using a biotin-conjugated cell-penetrating peptide.

The results are illustrated in FIG. 9. It was identified that in a case of using a cell-penetrating peptide conjugated with 25 nM streptavidin and 50 nM biotin, an antibody at 5 to 100 µg/mL (30 to 600 nM) was delivered into cells (FIG. 9: results obtained by performing flow cytometry analysis).

Then, it was identified using an antibody against BCl₂ whether the intracellularly delivered antibody was able to successfully label an intracellular target. The AF488-bound BCl₂ antibody was biotin-conjugated using EZ-Link^{™} Sulfo-NHS-LC-LC-Biotin, and then delivered using the same method. Mitochondria were labeled using mitotracker red. As a result, it was identified that there were many portions where green fluorescence exhibited by AF488 existed at the same position as red fluorescence exhibited by mitotracker (results not shown).

## Claims

1. A dimeric peptide, comprising, as a monomer, a peptide represented by Formula 1:
<Formula 1> X₁X₂X₃X₄X₅X₆X₇X₈X₉X₁₀
in the formula,
X₁, X₄, X₅, and X₈ are each independently a hydrophobic amino acid,
X₃, X₆, X₇, and X₁₀ are each independently a hydrophilic amino acid,
X₂ and X₉ are each independently an amino acid that forms a bond so that each monomeric peptide may be linked to each other at at least one position of X₂ and X₉ to form a dimeric peptide, and
X₁ is N-terminus and X₁₀ is C-terminus.

2. The dimeric peptide of claim 1, wherein X₁, X₄, X₅, and X₈ are each independently a hydrophobic amino acid selected from the group consisting of leucine (L), isoleucine (I), phenylalanine (F), valine (V), norvaline (norV), tryptophan (W), pentylglycine (pg), neopentylglycine (Npg), and cyclohexylalanine (Cha).

3. The dimeric peptide of claim 1, wherein X₃, X₆, X₇, and X₁₀ are each independently a hydrophilic amino acid selected from the group consisting of lysine (K), arginine (R), homoarginine (hR), norarginine (norR), histidine (H), omithine (O), diaminobutanoic acid (Dab), and diaminopropanoic acid (Dap).

4. The dimeric peptide of claim 1, wherein X₂ and X₉ are each independently an amino acid selected from the group consisting of cysteine (C), homocysteine (Hcy), penicillamine (Pen), selenocysteine (Sec, U), and leucine (L), provided that X₂ and X₉ are not leucine (L) at the same time.

5. The dimeric peptide of claim 1, wherein the bond formed between each monomeric peptide at at least one position of X₂ and X₉ is a covalent bond.

6. The dimeric peptide of claim 5, wherein the covalent bond is at least one selected from the group consisting of a disulfide bond, a diselenide bond, an ester bond, a maleimide bond, a thioester bond, a thioether bond, and a bond formed by a click reaction.

7. The dimeric peptide of claim 5, wherein the covalent bond is at least one selected from the group consisting of a disulfide bond between cysteine (C), homocysteine (Hcy), or penicillamine (Pen) residues, a diselenide bond between selenocysteine (See, U) residues, an ester bond, a maleimide bond formed by using a thiol functional group capable of participating in a reaction, a thioester bond, a thioether bond, and a bond formed by a click reaction in a case where triple bond- or azide group-containing non-natural amino acids, which are capable of causing a click reaction, are contained.

8. The dimeric peptide of claim 1, wherein a C₆ to C₁₂ fatty acid is bound to a position of X₁.

9. The dimeric peptide of claim 1, wherein X₁, X₄, X₅, and X₈ are each independently leucine (L), isoleucine (I), phenylalanine (F), valine (V), neopentylglycine (Npg), or cyclohexylalanine (Cha); X₃, X₆, X₇, and X₁₀ are each independently lysine (K), arginine (R), homoarginine (hR), norarginine (norR), histidine (H), omithine (O), diaminobutanoic acid (Dab), or diaminopropanoic acid (Dap); and X₂ and X₉ are each independently cysteine (C), homocysteine (Hcy), penicillamine (Pen), selenocysteine (Sec, U), or leucine (L), provided that X₂ and X₉ are not leucine (L) at the same time.

10. The dimeric peptide of claim 1, wherein the peptide is an amphipathic, alpha-helical peptide in the form of a homodimer or a heterodimer.

11. The dimeric peptide of claim 1, wherein each monomeric peptide is linked to each other in an anti-parallel direction.

12. The dimeric peptide of claim 1, wherein the monomer in the dimeric peptide has insignificant cell-penetrating ability but each monomer has a nanoparticle self-association characteristic in a case of being combined into a dimer; and the dimeric peptide exhibits cell-penetrating ability at a nanomolar concentration.

13. The dimeric peptide of claim 1, wherein the dimeric peptide has a ratio of cell-penetrating ability to cytotoxicity (IC₅₀⁻¹) at 62.5 nM, which is 130,000 or higher.

14. The dimeric peptide of claim 1, wherein the dimeric peptide has a ratio of nanoparticle self-association characteristic (ΔΔRt) to cytotoxicity (IC₅₀⁻¹) at 62.5 nM, which is 8 or higher.

15. The dimeric peptide of claim 1, wherein the peptide represented by Formula 1 comprises an amino acid sequence selected from the group consisting of SEQ ID NOs: 3 and 8 to 46.

16. A peptide represented by Formula 1:
<Formula 1> X₁X₂X₃X₄X₅X₆X₇X₈X₉X₁₀
in the formula,
X₁ to X₁₀ are each independently as defined in claim 1 or 9.

17. A pharmaceutical composition, comprising the dimeric peptide of any one of claims 1 to 15 and a biologically active substance.

18. The pharmaceutical composition of claim 17, wherein the biologically active substance is DNA, RNA, siRNA, an aptamer, a protein, an antibody, or a small molecule compound.

19. The pharmaceutical composition of claim 17, wherein the dimeric peptide and the biologically active substance are covalently linked to each other or exist in the form of a complex by being non-covalently linked to each other.

20. The pharmaceutical composition of claim 17, wherein the dimeric peptide is conjugated with biotin and streptavidin.
